# EUROPEAN PATENT APPLICATION

(11) **EP 0 614 981 A1**
(43) Date of publication of application: **14.09.1994**
(21) Application number: 94102452.3
(22) Date of filing: 18.02.1994
(51) Int. Cl.: C12N 15/62, C07K 13/00, C12P 21/08, C12N 5/10, A61K 37/02

(54) **Chimeric human interferon-gamma-receptor/immunoglobulin polypeptides**

(30) Priority: 05.03.1993 EP 93810170
(71) Applicant: F. HOFFMANN-LA ROCHE AG, CH-4002 Basel (CH)
(72) Inventor: Dembic, Zlatko, CH-4053 Basle (CH); Garotta, Gianni, CH-4153 Reinach (CH); Gentz, Reiner, Human Genome Sciences Inc., Rockville, MD 20850 (US)
(74) Representative: Mezger, Wolfgang, Dr.

(57) **Abstract**

The present invention relates to chimeric inferon-γ receptor/immunoglobulin polypeptides, to DNA sequences coding for these chimeric polypeptides and methods for making these.

SEQUENCE LISTING

(1) GENERAL INFORMATION:

(i) APPLICANT:
(A) NAME: F. HOFFMANN-LA ROCHE AG
(B) STREET: Grenzacherstrasse 124
(C) CITY: Basle
(D) STATE: BS
(E) COUNTRY: Switzerland
(F) POSTAL CODE (ZIP): CH-4002
(G) TELEPHONE: 061 - 688 42 56
(H) TELEFAX: 061 - 688 13 95
(I) TELEX: 962292/965542 hlr ch

(ii) TITLE OF INVENTION: Chimeric Human Interferon-Gamma
Receptor/Immunoglobulin Polypeptides

(iii) NUMBER OF SEQUENCES: 11

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(vi) PRIOR APPLICATION DATA:
(A) APPLICATION NUMBER: EP 93810170.6
(B) FILING DATE: 05-MAR-1993

(2) INFORMATION FOR SEQ ID NO: 1:

(i) SEQUENCE CHARACTERISTICS:
(A) LENGTH: 245 amino acids
(B) TYPE: amino acid
(D) TOPOLOGY: linear

(ii) MOLECULE TYPE: peptide

(iii) HYPOTHETICAL: NO

(iii) ANTI-SENSE: NO

(v) FRAGMENT TYPE: N-terminal

(xi) SEQUENCE DESCRIPTION: SEQ ID NO: 1:

Met Ala Leu Leu Phe Leu Leu Pro Leu Val Met Gln Gly Val Ser Arg
1               5                   10                  15

Ala Glu Met Gly Thr Ala Asp Leu Gly Pro Ser Ser Val Pro Thr Pro
            20                  25                  30

Thr Asn Val Thr Ile Glu Ser Tyr Asn Met Asn Pro Ile Val Tyr Trp
        35                  40                  45

Glu Tyr Gln Ile Met Pro Gln Val Pro Val Phe Thr Val Glu Val Lys
    50                  55                  60

Asn Tyr Gly Val Lys Asn Ser Glu Trp Ile Asp Ala Cys Ile Asn Leu
65                  70                  75                  80

Ser His His Tyr Cys Asn Ile Ser Asp His Val Gly Asp Pro Ser Asn
                85                  90                  95

Ser Leu Trp Val Arg Val Lys Glu Arg Val Gly Gln Lys Glu Ser Ala
            100                 105                 110

Tyr Ala Lys Ser Glu Glu Phe Ala Val Cys Arg Asp Gly Lys Ile Gly
        115                 120                 125

Pro Pro Lys Leu Asp Ile Arg Lys Glu Glu Lys Gln Ile Met Ile Asp
    130                 135                 140

Ile Phe His Pro Ser Val Phe Val Asn Gly Asp Glu Gln Glu Val Asp
145                 150                 155                 160

Tyr Asp Pro Glu Thr Thr Cys Tyr Ile Arg Val Tyr Asn Val Tyr Val
                165                 170                 175

Arg Met Asn Gly Ser Glu Ile Gln Tyr Lys Ile Leu Thr Gln Lys Glu
            180                 185                 190

Asp Asp Cys Asp Glu Ile Gln Cys Gln Leu Ala Ile Pro Val Ser Ser
        195                 200                 205

Leu Asn Ser Gln Tyr Cys Val Ser Ala Glu Gly Val Leu His Val Trp
    210                 215                 220

Gly Val Thr Thr Glu Lys Ser Lys Glu Val Cys Ile Thr Ile Phe Asn
225                 230                 235                 240

Ser Ser Ile Lys Gly
                245

(2) INFORMATION FOR SEQ ID NO: 2:

    (i) SEQUENCE CHARACTERISTICS:
        (A) LENGTH: 735 base pairs
        (B) TYPE: nucleic acid
        (C) STRANDEDNESS: single
        (D) TOPOLOGY: linear

   (ii) MOLECULE TYPE: DNA (genomic)

  (iii) HYPOTHETICAL: NO

  (iii) ANTI-SENSE: NO

 (viii) POSITION IN GENOME:
        (C) UNITS: bp

(xi) SEQUENCE DESCRIPTION: SEQ ID NO: 2:

ATGGCTCTCC TCTTTCTCCT ACCCCTTGTC ATGCAGGGTG TGAGCAGGGC TGAGATGGGC 60

ACCGCGGATC TGGGGCCGTC CTCAGTGCCT ACACCAACTA ATGTTACAAT TGAATCCTAT 120

AACATGAACC CTATCGTATA TTGGGAGTAC CAGATCATGC CACAGGTCCC TGTTTTTACC 180

GTAGAGGTAA AGAACTATGG TGTTAAGAAT TCAGAATGGA TTGATGCCTG CATCAATCTT 240

TCTCATCATT ATTGTAATAT TTCTGATCAT GTTGGTGATC CATCAAATTC TCTTTGGGTC 300

AGAGTTAAAG AAAGGGTTGG ACAAAAAGAA TCTGCCTATG CAAAGTCAGA AGAATTTGCT 360

GTATGCCGAG ATGGAAAAAT TGGACCACCT AAACTGGATA TCAGAAAGGA GGAGAAGCAA 420

ATCATGATTG ACATATTTCA CCCTTCAGTT TTTGTAAATG GAGACGAGCA GGAAGTCGAT 480

TATGATCCCG AAACTACCTG TTACATTAGG GTGTACAATG TGTATGTGAG AATGAACGGA 540

AGTGAGATCC AGTATAAAAT ACTCACGCAG AAGGAAGATG ATTGTGACGA GATTCAGTGC 600

CAGTTAGCGA TTCCAGTATC CTCACTGAAT TCTCAGTACT GTGTTTCAGC AGAAGGAGTC 660

TTACATGTGT GGGGTGTTAC AACTGAAAAG TCAAAAGAAG TTTGTATTAC CATTTTCAAT 720

AGCAGTATAA AAGGT 735

(2) INFORMATION FOR SEQ ID NO: 3:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: DNA (genomic)

(iii) ANTI-SENSE: YES

(viii) POSITION IN GENOME:
(C) UNITS: bp

(xi) SEQUENCE DESCRIPTION: SEQ ID NO: 3:

AATTCGAGCT CGTAGCAGCA TGGCTCTCCT CTTTC 35

(2) INFORMATION FOR SEQ ID NO: 4:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: YES

(viii) POSITION IN GENOME:
(C) UNITS: bp

(xi) SEQUENCE DESCRIPTION: SEQ ID NO: 4:

TTAAGCTTAC TTACCACCTT TTATACTGCT ATTGA 35

(2) INFORMATION FOR SEQ ID NO: 5:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: YES

(viii) POSITION IN GENOME:
(C) UNITS: bp

(xi) SEQUENCE DESCRIPTION: SEQ ID NO: 5:

AATTCGAGCT CACCTTTTAT ACTGCTATTG A 31

(2) INFORMATION FOR SEQ ID NO: 6:

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: YES

(viii) POSITION IN GENOME:
(C) UNITS: bp

(xi) SEQUENCE DESCRIPTION: SEQ ID NO: 6:

CCGCTCGAGC GG                                                    12

(2) INFORMATION FOR SEQ ID NO: 7:

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: YES

(viii) POSITION IN GENOME:
(C) UNITS: bp

(xi) SEQUENCE DESCRIPTION: SEQ ID NO: 7:

CCGCTCGAGC GG                                                    12

(2) INFORMATION FOR SEQ ID NO: 8:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(viii) POSITION IN GENOME:
(C) UNITS: bp

(xi) SEQUENCE DESCRIPTION: SEQ ID NO: 8:

GGGCTCGAGA CCTTATGGGA CTTTCCTACT TGG                             33

(2) INFORMATION FOR SEQ ID NO: 9:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(viii) POSITION IN GENOME:
(C) UNITS: bp

(xi) SEQUENCE DESCRIPTION: SEQ ID NO: 9:

CCCGTCGACC CTACCGCCCA TTTGCGTCAA TG 32

(2) INFORMATION FOR SEQ ID NO: 10:

(i) SEQUENCE CHARACTERISTICS:
(A) LENGTH: 41 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(viii) POSITION IN GENOME:
(C) UNITS: bp

(xi) SEQUENCE DESCRIPTION: SEQ ID NO: 10:

AAGCTTGGCC AGGATCCAGC TGACTGACTG ATCGCGAGAT C 41

(2) INFORMATION FOR SEQ ID NO: 11:

(i) SEQUENCE CHARACTERISTICS:
(A) LENGTH: 41 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(viii) POSITION IN GENOME:
(C) UNITS: bp

(xi) SEQUENCE DESCRIPTION: SEQ ID NO: 11:

GATCTCGCGA TCAGTCAGTC AGCTGGATCC TGGCCAAGCT T 41

## Description

Interferon-γ (IFNγ) is a protein produced by NK cells and activated helper T lymphocytes which has antiviral and antiproliferative activity and which plays a multipotent role in the control of the immune system and of the inflammatory response. It regulates antibodies formation and T lymphocytes or NK cells differentiation. It enhances the expression of Major Histocompatibility Complex (MHC) class I and II structures on several cell types, amplifying their antigen presentation (accessory cells) capacity. Acting on phagocytes, fibroblasts, epithelial and endothelial cells, IFNγ plays a central role in the activation of non-specific defense mechanisms. It is the main activator of microbicidal and tumoricidal properties of macrophages, enhances phagocytosis, induces ADCC (Antibody Dependent Cellular Cytotoxicity) and modulates the release of polypeptides encoded by MHC class III genes (i.e. complement components and TNFα), proteinase inhibitors (C1 inhibitor), IL1, GM-CSF, fibronectin, arachidonic acid metabolites, 1,25-hydroxy-vitamin D3, lysosomal enzymes (IP-30, hydrolases, neutral proteinases, i:e: uPA), and chemotactic factors (IP-10). Finally, IFNγ promotes cell-to-cell interaction, migration of phagocytes through connective tissue, chemotaxis and adhesion to extracellular matrix glycoproteins (Landolfo and Garotta, J. Immunol. Res. 3, 81-94 [1991]).

Several animal experiments suggest that IFNγ plays a crucial role in either the induction or the progression of insulin-dependent diabetes, systemic lupus erythematosus, thyroiditis, multiple sclerosis, fulminant hepatitis, allograft rejection, thrombosis and hemorrhage that follows the generalized Shwartzman-type reaction. Additionally, IFNγ plays a role in the progression of Kawasaki disease (mucocutaneous lymph node syndrome) and in the hypercalcemia observed in some of sarcoidosis patients (IFNγ induces the elevation of l,a-sterol-hydroxylase that converts vitamin D3 into its most active form 1,25-dihydroxy-vitamin D3) (Garotta et al., Pharmacol. Res. 21, 5-17 [1989]; Landolfo and Garotta, supra; Gilles et al., Hepatology 16, 655-663 [1992]). In the pathogenesis of AIDS, tissue macrophages play a role in the establishment of a state of chronic infection because they represent an important reservoir for human immunodeficiency virus (HIV). In vitro experiments show that IFNγ enhances the expression of mature HIV by infected macrophages and can exacerbate the progression of the disease (Ganser et al., Onkologie 9, 163-166 [1986], Biswas et al., J. Exp. Med., 176, 739-750 [1992]). Finally, IFNγ plays an important role in inflammatory neurological diseases or in neurological complications of AIDS, poliovirus infections, Lyme disease and septicemia. IFNγ activated macrophages convert L-tryptophan into the neurotoxin quinolinic acid (Heyes et al., Biochem. J. 283, 633-635 [1992]).

The existence of a specific receptor for IFNγ (IFNγR) on various cells was demonstrated by cross-linking experiments, by binding of radiolabeled IFNγ and by specific competition with unlabeled IFNγ. Cross-linked complexes between human IFNγ (hIFNγ) and human IFNγ receptor (hIFNγR) with a molecular weight (Mr) ranging from 70,000 to 165,000 have been described (Rubinstein et al., Immunol. Rev. 97, 29-50 [1987]). Despite this apparent heterogeneity, binding and structural studies on different cell types revealed only one IFNγ binding protein with 90 kDa of apparent Mr (p90) and a single class of binding site with a dissociation constant of about 10⁻¹¹ to 10⁻¹⁰ M (Sarkar and Gupta, Proc. Natl. Acad. Sci. USA 81, 5160-5164 [1984]; Aguet and Merlin, J. Exp. Med. 165, 988-999 [1987]; Calderon et al., Proc. Natl. Acad. Sci. USA 85, 4837-4841 [1988]; Fountoulakis et al., J. Immunol., 143, 3266-3276 [1989]; van Loon A.P.G.M. et al., J. Leukocyte Biol., 49, 462-473 [1991]). An additional 50 Kd (p50) component of hIFNγR that can be detected is a proteolytic degradation product of the p90 protein (Aguet and Merlin, supra, Sheehan et al., J. Immunol. 140, 4231-4237 [1988]; Fountoulakis et al., supra; van Loon A.P.G.M. et al., supra). Since this P50 component of hIFNγR includes the extracellular domain, the transmembrane region and a portion of the intracellular domain, it is not a natural occurring soluble form of hIFNγR (Fountoulakis et al., supra; van Loon A.P.G.M. et al., supra). In general, the IFNγR is an ubiquitous membrane anchored protein (Valente et al., Eur. J. Immunol. 22, 2403-2412 [1992]) that seems to be expressed to a lesser extent on normal cells (up to 10³ sites per cell) than on tumor cells. Thus, some human carcinoma and B-cell lines were reported to express in the order of 10⁴ binding sites per cell (Uecer et al., Cancer Res. 46, 5339-5343 [1986]; Aguet and Merlin, supra).

Molecular cloning and expression of the hIFNγR has been described (Aguet et al., Cell 55, 273-280 [1988]). However, because the natural IFNγR as well as the recombinant IFNγR are membrane anchored proteins, they have the disadvantage that they are insoluble in physiological solution. Consequently, search or design of an IFNγ antagonist, and administration of purified IFNγR to mammals to inhibit IFNγ binding to its specific receptor thereby preventing, suppressing and/or modulating the course of autoimmune disorders, chronic inflammations, delayed hypersensitivities and allotransplant rejections was difficult to accomplish.

The soluble forms of human [shIFNγR] and mouse IFNγR [smIFNγR] were engineered by culturing transformants carrying an expression vector containing a DNA sequence coding for a soluble form of the respective IFNγR and isolating such a soluble IFNγR. The said shIFNγR includes the whole extracellular domain of the natural IFNγR from the N-terminal portion to the transmembrane region (at least amino acids 26-246 of the natural IFNγR sequence), lacks the cytoplasmic and transmembrane domains of the natural IFNγ receptor and is capable of specifically binding IFNγ (Fountoulakis et al., J. Biol. Chem. 265, 13268-13275 [1990], Gentz et al., Eur. J. Biochem. 210, 545-554 [1992] and European Patent Application, Publication No. 393 502). This soluble form of the IFNγR has a molecular mass of about 30-32 kDa, binds IFNγ with an affinity of about 10⁻¹⁰ M, is active and stable in vivo, is not immunogenic and can be applied as a drug that specifically neutralizes the endogenous IFNγ. Accordingly, it can be used for the therapy of insulin-dependent diabetes, systemic lupus erythematosus, multiple sclerosis, fulminant hepatitis, thyroiditis, allowgraft rejection, septic peritonotis and Shwartzman-type reaction and inflammatory neurological diseases produced by the neurotoxin quinolinic acid released by IFNγ activated macrophages. Additionally, the soluble form of the IFNγR can be applied to AIDS patients to delay the chronicization of the infection, to prolong the asymptomatic phase of the disease and to prevent neurological complications. The soluble form of IFNγ receptor can be applied as IFNγ antagonist to prevent neurological complications of poliovirus infections, Lyme disease and septicemia. However, the soluble form of the IFNγR shows a persistency of only 1-3 hours in the blood and of 6 hours in the lymphoid organs (Ozmen et al., J. Chemotherapy 3, Suppl. 3, 99-102 [1991]; Ozmen et al., J. Immunol. Methods 147, 261-270 [1992]; Gentz et al, supra).

By combining the soluble form of the mouse IFNγR with parts of the constant domain of mouse immunoglobulins resulting in chimeric mouse IFNγR-immunoglobulin polypeptides (Kürschner et al., J. Biol. Chem. 267, 9354-9360 [1992]) increased mouse IFNγR persistency in the blood and the lymphoid organs could be achieved (Kürschner et al., J. Immunol. 149, 4096-4100 [1992]).

These chimeric mouse IFNγR-immunoglobulin polypeptides have, however, the disadvantage that they cannot be used for administration to humans to inhibit IFNγ binding to its specific receptor. To solve this problem DNA sequences coding for chimeric human IFNγR-human immunoglobulin polypeptides have been constructed.

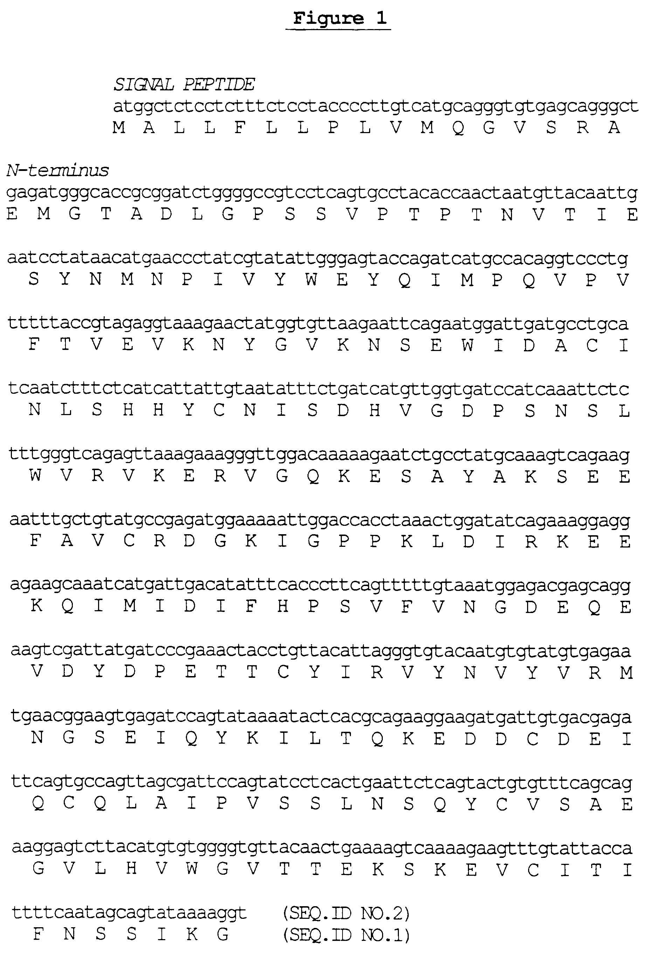

More precisely, the present invention provides DNA sequences comprising a combination of two partial DNA sequences, wherein one of said partial sequences is coding for a fragment of the hIFNγR binding hIFNγ, whereby a fragment of the hIFNγR with the whole or a part of the sequence as shown in Figure 1 is preferred, and the other partial sequence is coding for part or all of the constant domains of human immunoglobulin heavy or light chains, whereby heavy chains, especially all domains except the first domain of the constant domain of human immunoglobulins, such as IgG, IgA, IgM or IgE and specifically IgG, e.g. IgG1 and IgG3, are preferred. A preferred constant domain of human immunoglobulin light chains is, e.g. Cχ.

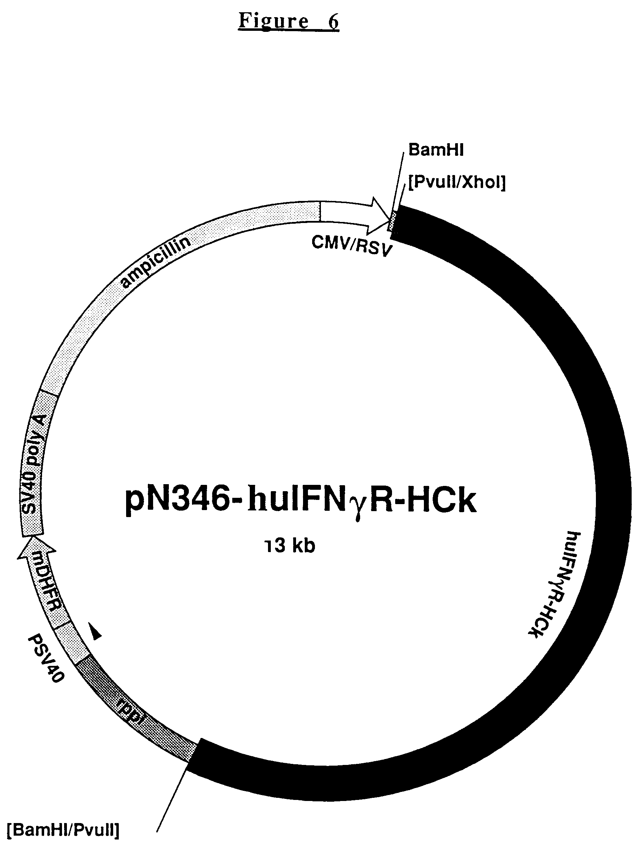

The present invention is also concerned with the recombinant chimeric polypeptides coded by such DNA sequences, such as hIFNγR-HG1, hIFNγR-HG3 or hIFNγR-HCχ. The chimeric polypeptides may contain amino acid substitutions that do not significantly change the activity of the proteins. Amino acid substitutions in proteins and peptides which do not generally alter the activity of such molecules are known in the art and are described, e.g. by H. Neurath and R.L. Hill in "The Proteins" (Academic Press, New York, 1979, see especially Figure 6, page 14). The most commonly occurring exchanges are: Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly and vice versa.

The recombinant chimeric polypeptides of the present invention can additionally contain sequences of several amino acids which are coded for by "linker" sequences. These sequences arise as a result from the expression vectors used for expression of the recombinant chimeric polypeptides.

The recombinant chimeric polypeptides of the present invention can also contain specific sequences that perferably bind to an affinity carrier material. Examples of such sequences are sequences containing at least two adjacent histidine residues (see in this respect European Patent Application Publication No. 282 042). Such sequences bind selectively to nitrilotriacetic acid nickel chelate resins (Hochuli and Döbeli, Biol. Chem. Hoppe-Seyler 368, 748 (1987); European Patent No. 253 303). Recombinant chimeric polypeptides which contain such a specific sequence can, therefore, be separated selectively from the remaining polypeptides. The specific sequence can be linked either to the C-terminus or the N-terminus of the amino acid sequence of the chimeric polypeptide.

Such chimeric polypeptides could have increased half-life in vivo. Increased half-life in vivo has been shown, e.g., for chimeric polypeptides consisting of the first two domains or parts thereof of the human CD4-molecule and different domains of the constant regions of the heavy chain or the light chain of a mammalian immunoglobulin (see Traunecker et al., Nature 331, 84-86 [1988] and European Patent Application, Publication No. 394 827). Additionally, as already mentioned hereinbefore, chimeric mouse IFNγR-immunoglobulin polypeptides show increased half-life in the blood and in the lymphoid organs.

Because the complete DNA sequence of the gene coding for the natural IFNγR is known (Aguet et al., supra) a DNA sequence coding for a fragment of the hIFNγR can be chemically synthesized using standard methods known in the art, preferably solid state methods, such as the methods of Merrifield (J. Am. Chem. Soc. 85, 2149-2154 [1963]). Alternatively, fragments of the hIFNγR can be produced from DNA encoding the hIFNγR using methods of DNA recombinant technology (Sambrook et al. in "Molecular Cloning-A Laboratory Manual", 2nd. ed., Cold Spring Harbor Laboratory [1989]).

Preferably, fragments are prepared including the signal sequence and the extracellular portion of the hIFNγR by polymerase chain reaction (PCR) using plasmids encoding the hIFNγR as described in detail in Examples 5, 6 and 7.

Plasmids suitable for amplification of DNA sequences coding for the IFNγR by PCR are described, for example, in European Patent Appliation, Publication No. 393 502. An especially suitable plasmid is plasmid phIFNγR (Example 2).

The DNA sequences coding for fragments of the hIFNγR can then be integrated into suitable expression vectors containing DNA sequences coding for part or all of the constant domains of human immunoglobulin (Ig) heavy or light chains using known methods (Sambrook et al., supra). The Ig constant domains can be provided by expression vectors which have been used to express CD4 as hybrid molecules with Ig constant domains. Such expression vectors include but are not limited to pSV2-derived vectors (see for example German, C. in "DNA Cloning", Vol. II., edt. by Glover, D.M., IRL Press, Oxford, 1985), like pCD4-Hµ, pCD4-Hγ1, pCD4-Hγ3 (described in detail in European Patent Application, Publication No. 394 827).

The specification of European Patent Application, Publication No. 394 827 and the Traunecker et al. reference cited supra contain also data with respect to the further use of these vectors for the expression of chimeric proteins and for the construction of vectors for the expression of chimeric proteins with other immunoglobulin fragments. For the purpose of the present invention the CD4 coding part in these vectors is replaced by a DNA sequence coding for a fragment of the hIFNγR by methods known in the art and described, e.g., in Sambrook et al. (supra), resulting, for example, in plasmids phuIFNγR-HG1 (Example 5), phuIFNγR-HG3 (Example 6) and phuIFNγR-HCχ (Example 7).

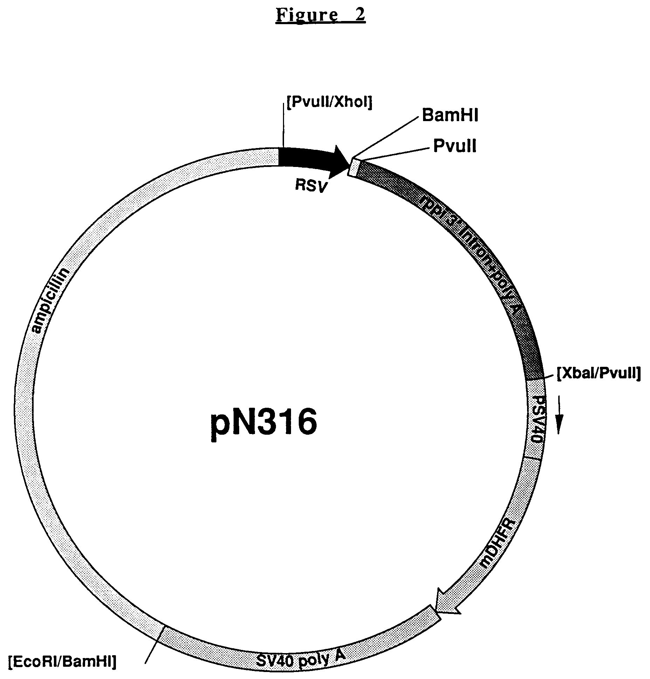

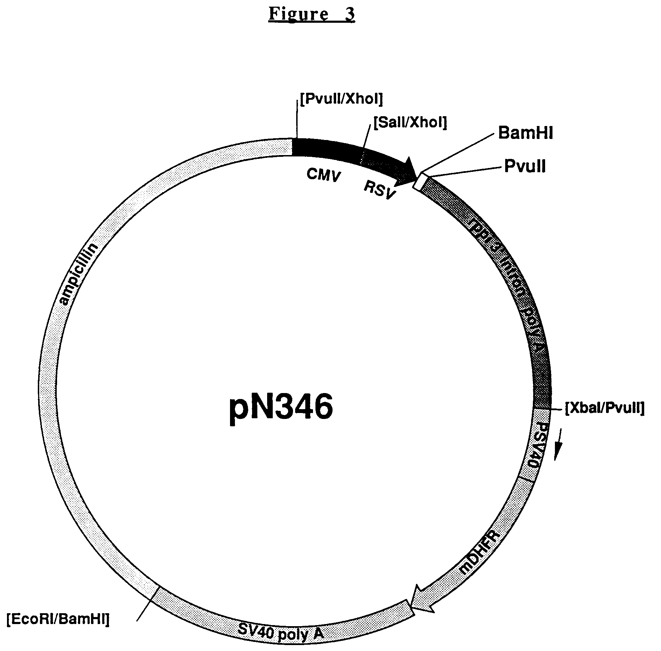

Suitable expression vectors include, for example, vectors such as pBC12MI [ATCC 67109], pSV2dhfr [ATCC 37146], pSVL [Pharmacia, Uppsala, Sweden], pRSVcat [ATCC 37152] and pMSG [Pharmacia, Uppsala]. Preferred vectors for the expression of the chimeric hIFNγR-immunoglobulin polypeptides are pN316 and pN346 type vectors (see Figures 2 and 3) resulting in expression vectors pN316-huIFNγR-HG1, pN316-huIFNγR-HG3 and pN346-huIFNγR-HCχ (see Examples 5, 6 and 7), which have been deposited transformed in E. coli K803 under the conditions of the Budapest Treaty for patent purposes at the Deutsche Sammlung von Mikroorganismen und Zellkulturen GmbH (DSM) in Braunschweig, Federal Republic of Germany on January 26, 1993, under accession numbers DSM 7421, 7419, 7420 respectively. These vectors are preferably introduced into suitable mammalian host cells, for example, by transfection.

Mammalian host cells that could be used include, e.g., human Hela, H9 and Jurkat cells, mouse NIH3T3 and C127 cells, CV1 African green monkey kidney cells, quail QC1-3 cells, Chinese hamster ovary (CHO) cells, mouse L cells and the COS cell lines. The CHO cell line (ATCC CCL 61) is preferred.

The DNA sequences coding for the chimeric hIFNγR-immunoglobulin polypeptides can also be integrated into suitable vectors for expression in yeast or insect cells. For the production in insect cells, e.g., the baculovirus-insect cell vector system can be used (for review see Luclow and Summers, Bio/Technology 6, 47-55 [1988]). The chimeric polypeptides produced in insect cells infected with recombinant baculovirus can undergo post-translational processing including N-glycosylation (Smith et al., Proc. Nat. Acad. Sci. USA 82, 8404-8408) and O-glycosylation (Thomsen et al., 12. International Herpesvirus Workshop, University of Philadelphia, Pennsylvania).

The manner in which the expression of the chimeric hIFNγR-immunoglobulin polypeptides of the present invention is carried out depends on the chosen expression vector/host cell system.

Usually, mammalian host cells which contain a desired expression vector are grown under conditions which are optimal for the growth of the mammalian host cells. A typical expression vector contains the promoter element, which mediates the transcription of mRNA, the protein coding sequence, and the signals required for efficient termination and polyadenylation of the transcript. Additional elements may include enhancers and intervening sequences bounded by spliced donor and acceptor sites.

Most of the vectors used for the transient expression of a given coding sequence carry the SV40 origin of replication, which allows them to replicate to high copy numbers in cells (e.g. COS cells) that constitutively express the T antigen required to initiate viral DNA synthesis. Transient expression is not limited to COS cells. Any mammalian cell line that can be transfected can be utilized for this purpose. Elements that control a high efficient transcription include the early or the late promoters from SV40 and the the long terminal repeats (LTRs) from retroviruses, e.g. RSV, HIV, HTL VI. However, also cellular signals can be used (e.g. human-β-actin-promoter).

Alternatively, stable cell lines carrying a gene of interest integrated into the chromosome can be selected upon co-transfection with a selectable marker such as gpt, dhfr, neomycin or hygromycin.

Now, the transfected gene can be amplified to express large quantities of a foreign protein. The dihydrofolate reductase (DHFR) is a useful marker to develop lines of cells carrying more than 1000 copies of the gene of interest. The mammalian cells are grown in increasing amounts of methotrexate. Subsequently, when the methotrexate is withdrawn, cell lines contain the amplified gene integrated into the chromosome. In the expression vectors used in the preferred embodiments of the present invention, the expression is controlled by the Rous sarcoma virus LTR promoter.

The host cells transfected with a suitable expression vector as well as the expression vectors used for their transfection and expressing the recombinant chimeric polypeptides are also an object of the present invention.

The chimeric polypeptides of the present invention can be purified from the cell mass or the culture supernatants according to methods of protein chemistry which are known in the art such as, for example, precipitation, e.g., with ammonium sulfate, dialysis, ultrafiltration, gelfiltration, ion-exchange chromatography, SDS-PAGE, isoelectric focusing, affinity chromatography like immunoaffinity chromatography, HPLC on normal or reverse systems or the like. Preferably, the chimeric hIFNγR-immunoglobulin polypeptides expressed in mammalian host cells are obtained after affinity chromatography.

The chimeric polypeptides of the present invention as well as their physiologically compatible salts, can be used for the treatment of illnesses in which IFNγ is involved in their course, e.g., for the treatment of autoimmune diseases, e.g. type I diabetes or lupus erythematosus or rheumatoid arthritis, chronic inflammations, e.g. Shwartzman Reaction, delayed hypersensitivity, allotransplant rejections, multiple sclerosis, fulminant hepatitis and inflammatory neurological diseases produced by the neurotoxin quinolinic acid released by IFNγ activated macrophages. Additionally, the chimeric polypeptides can be applied to AIDS patients to delay the chronicization of the infection, to prolong the asymptomatic phase of the disease and to prevent neurological complications. The chimeric polypeptides can be also used as IFNγ antagonist to prevent neurological complications of poliovirus infections, Lyme disease and septicemia and/or the production of corresponding pharmaceutical preparations. They may be administered in pharmaceutically acceptable oral, injectable or topical compositions and modes. Dosage and dose rate may parallel that currently being used in clinical applications of the known IFNs. The pharmaceutical compositions of the present invention contain the chimeric polypeptides or their physiologically compatible salts thereof in association with a compatible pharmaceutically acceptable carrier material. Any conventional carrier material can be utilized. The carrier material can be an organic or inorganic one suitable for enteral, percutaneous or parenteral administration. Suitable carriers include water, gelatine, gum arabic, lactose, starch, magnesium stearate, talc, vegetable oils, polyalkylene-glycols, petroleum jelly and the like. Furthermore, the pharmaceutical preparations may contain other pharmaceutically active agents. Additional additives such as flavouring agents, preservatives, stabilizers, emulsifying agents, buffers and the like may be added in accordance with accepted practices of pharmaceutical compounding.

The pharmaceutical preparations can be made up in any conventional form including: a) a solid form for oral administration such as tablets, capsules, pills, powders, granules and the like; b) a liquid form for oral administration such as solutions, syrups, suspensions, elixirs and the like; c) preparations for parenteral administration such as sterile solutions, suspensions or emulsions; and d) preparations for topical administrations such as solutions, suspensions, ointments, creams, gels, micronized powders, aerosols and the like. The pharmaceutical preparations may be sterilized and/or may contain adjuvants such as preservatives, stabilizers, wetting agents, emulsifiers, salts for varying the osmotic pressure and/or buffers.

Parenteral dosage forms may be infusions or injectable solutions which can be injected intravenously or intramuscularly. These preparations can also contain other medicinally active substances. Additional additives such as preservatives, stabilizers, emulsifying agents, buffers and the like may be added in accordance with accepted practices of pharmaceutical compounding.

Such pharmaceutical preparations and the use of the compounds of the present invention for therapeutical purposes are also an object of the present invention.

Having now generally described this invention, the same will become better understood by reference to the specific examples, which are included herein for purpose of illustration only and are not intended to be limited unless otherwise specified, in connection with the following figure:
Figure 1 displays the nucleic acid sequence of shIFNγR cDNA (SEQ. ID No.2) and the amino acid sequence deduced therefrom (SEQ. ID No. 1). Sequences are represented by standard abbreviations for nucleotides and amino acids.
Figure 2 is a schematic drawing of the expression vector pN316. The abbreviations and symbols used are: RSV = rous sarcoma virus LTR sequence (promoter); rppi 3' intron + poly A = rat preproinsulin 3' intron and polyadenylation site; PSV 40 = SV40 viral promoter; mDHFR = mouse DHFR; SV40 poly A = SV40 virus polyadenylation site sequence; ampicillin = β-lactamase gene that confers resistance to the antibiotic. Restriction enzyme sites which are inactivated by the subcloning procedures described in the Examples are indicated in brackets.
Figure 3 is a schematic drawing of the expression vector pN346. The abbreviations and symbols used are: RSV = rous sarcoma virus LTR sequence (promoter); rppi 3' intron + poly A = rat preproinsulin 3' intron and polyadenylation site; PSV40 = SV40 viral promoter; mDHFR = mouse DHFR; SV40 poly A = SV40 virus polyadenylation site sequence; ampicillin =β-lactamase gene that confers resistance to the antibiotic; CMV = part of Cytomegalovirus enhancer sequence. Restriction enzyme sites which are inactivated by the subcloning procedures described in the Examples are indicated in brackets.
Figure 4 is a schematic drawing of the expression vector pN316-huIFNγR-HG1. For abbreviations and symbols see legend to Fig. 2.
Figure 5 is a schematic drawing of the expression vector pN316-huIFNγR-HG3. For abbreviations and symbols see legend to Fig. 2.
Figure 6 is schematic drawing of the expression vector pN346-huIFNγR-HCχ. For abbreviations and symbols see legend to Fig. 3.
Figure 7 displays standard curves for shIFNγR and hIFNγR-HG3.

### Example 1

### Sequencing

All cDNAs or fragments thereof obtained by suitable restriction enzyme digests were subcloned in pUC18/19 type vectors (Pharmacia, Uppsala, Sweden). Sequencing was performed using a protocol based on the Sanger procedure and involving Taq polymerase and double stranded DNA.

### Example 2

### Construction of expression vectors pN346 and pN316

1. Preparation of the fragment containing part of the CMV enhancer.
   A fragment containing 232 basepairs of the enhancer of the immediate early gene of human cytomegalovirus (CMV) (Boshart et al., Cell 41, 521-530 [1985]) was obtained using the Polymerase Chain Reaction (PCR) (Saiki et al., Science 230, 1350-1354 [1985]).
   PCR is based on the enzymatic amplification of a DNA fragment: Two oligonucleotide primers that are oriented with their 3' ends towards each other are hybridized to opposite strands of the target sequence.
   Repeated cycles of heat denaturation of the template, annealing of the primers to their complementary sequences and extension of the annealed primers with a DNA polymerase result in the amplification of the segment defined by the 5' ends of the PCR primers. The addition of restriction enzyme sites to the 5' end of each primer facilitates cloning of the final PCR product (Scharf et al., Science 233, 1076-1078 [1986]).
   The following oligonucleotides have been used to amplify the DNA fragment containing part of the CMV enhancer:
   1) 5'-GGG*CTCGAG* ACCTTATGGGACTTTCCTACTTGG 3' (SEQ. ID No. 8) (forward primer), and
   2) 5'-CCC*GTCGAC* CCTACCGCCCATTTGCGTCAATG 3' (SEQ. ID No. 9) (reverse primer).
   The amplified fragment was isolated from an agarose gel using a commercial available kit ("Geneclean", BIO 101 Inc., La Jolla, Ca.). The fragment was then digested with the endonucleases Xhol and Sall (restriction sites in the above primers underlined) and then purified again as described above (Fragment 1).
2. Preparation of the vector fragment
   The expression vector pK21 which contains the strong promoter of the long terminal repeat (LTR) of the Rous Sarcoma Virus (Cullen et al., Molecular and Cellular Biology, March 1985, 438-447) and a polylinker region of the following sequence: 
5'-AAGCTTGGCCAGGATCCAGCTG ACTGACTGATCGCGAGATC3' (SEQ.ID No. 10)
3'-TTCGAACCGGTCCTAGGTCGAC TGACTGACTAGCGCTCTAG5' (SEQ.ID No. 11)
 that allows the integration of genes of interest (PvuII site underlined) was used. Downstream the cloning sites for the genes this vector contains the 3' intron, the polyadenylation site and termination signal of the rat preproinsulin gene.
   Plasmid pK21 was digested with XhoI and then treated with calf intestinal alkaline phosphatase to remove the phosphates from the 5' ends of the DNA fragments. The dephoshorylated vector was then isolated from an agarose gel as described above (Fragment V1).
3. Preparation of plasmid pN340
   Fragment 1 was ligated with fragment V1 using T4 ligase.
   E.coli HB101 cells were then transformed and transformants which contained the enhancer fragment in the proper orientation (same orientation as in the CMV enhancer) were identified by restriction enzyme mapping and sequencing. The resulting plasmids were named pN340.
4. Construction of the expression vector pN346.
   Preparation of fragment F2
   Plasmid pN340 was digested with Xhol and Xbal. The XhoI and XbaI fragment contains the promoter (CMV-enhancer fragment plus RSV-LTR), the polylinker region shown above and the 3' intron plus the polyadenylation signal of the rat preproinsulin gene. The fragment was isolated from an agarose gel as described above using Genclean. The sticky ends of this fragment were then filled in using Klenow enzyme (Fragment 2).
   Preparation of the vector fragment V2.
   Plasmid pN308 which is a derivative of the expression vector pSV2-DHFR (supra). missing the region between the single EcoRI and BamHI restriction sites was digested with PvuII and then treated with calf intestinal alkaline phosphatase. The dephosphorylated vector was then isolated as described (Fragment V2).
   Ligation of fragment F2 and vector V2.
   The blunt ended vector fragment V2 and the blunt ended fragment F2 were ligated with T4 ligase.
   E.coli HB 101 cells were then transformed and transformants which contained the Fragment 2 in the desired orientation were identified by restriction enzyme analysis and sequencing. The resulting plasmids were named pN346 (Figure 3).
5. Construction of the plasmid pN316
   The construction of plasmid pN316 was the same as described under paragraph 4 above with the exception that instead of plasmid pN340, plasmid pK21 was used. As a result, plasmid pN316 lacks Fragment 1 (described in paragraph 1) and has only the RSV LTR promoter element (Figure 2).

### Construction of plasmid "phIFNγR"

Plasmid constructions were carried out as described in the following paragraphs. In case that no specific references or details of preparation are given standard methodology according to Sambrook et al. in "Molecular Cloning - A Laboratory Manual" (2nd ed.), Cold Spring Harbor Laboratory [1989], was used.

The insert from phage λgtll-hIFNγR-16 (Aguet et al., Cell 55, 273-280 [1988]) was excised using EcoRl restriction enzyme under conditions where a partial digestion of the DNA was obtained. The complete insert of about 2,2 kb was ligated into plasmid pUC18 and one of the resulting constructs, plasmid phIFNγR, was chosen for further analysis.

### Example 3

### Transformation of E.coli K803

Transformation of E.coli K803 cells with plasmid phIFNγR (Example 2) was achieved by the heat shock procedure as described in Sambrook et al., supra.

### Example 4

### Isolation of Plasmid DNA

Plasmid DNA from transformed E.coli K803 cells (Example 3) was prepared using a standard procedure /Birnboim and Doly, Nucl. Acids Res. 7, 1513 (1979); Sambrook et al., 1989, supra). The insert coding for soluble hIFNγR was cut out of plasmid phIFNγR and sequenced as described in Example 1. The complete nucleic acid sequence of shIFNγR and the amino acid sequence deduced therefrom are shown in Fig. 1.

### Example 5

### Construction of a chimeric human IFNγR-InGl (HG 1) molecule

In the first step, a polymerase chain reaction (PCR) was performed using plasmid phIFNγR as a template and the following primers:
1) 5'-AATTCGAGCTCGTAGCAGCATGGCTCTCCTCTTTC 3' (SEQ. ID NO.3) matching 24 nucleotides of the hIFNγR cDNA sequence (8 residues of the 5' untranslated (5'-UT) and 16 nucleotides of the hIFNγR coding region), containing a SacI restriction enzyme site, and
2) 5'-TTAAGCTTACTTACC ACCTTTTATACTGCTATTGA-3' (SEQ. ID. NO.4) matching the last 20 nucleotides of the coding region of shIFNγR and containing in addition 15 residues among which three nucleotides code for the first amino acid residue of the hinge region in the HGγ1 molecule, and the others a splicing site followed by a HindIII recognition site.

The PCR was performed using Taq Polymerase, under conditions as described by the manufacturer (Perkin-Elmer, Cetus, USA).

After phenol extraction and ethanol precipitation, the PCR product was resuspended in an appropriate buffer, and was digested by the SacI and HindIII restriction enzymes by methods described in the art.

The PCR fragment was then ligated into SacI-HindIII digested and gel purified pCD4-Hγ1 vector generating plasmid phuIFNγR-HG1.

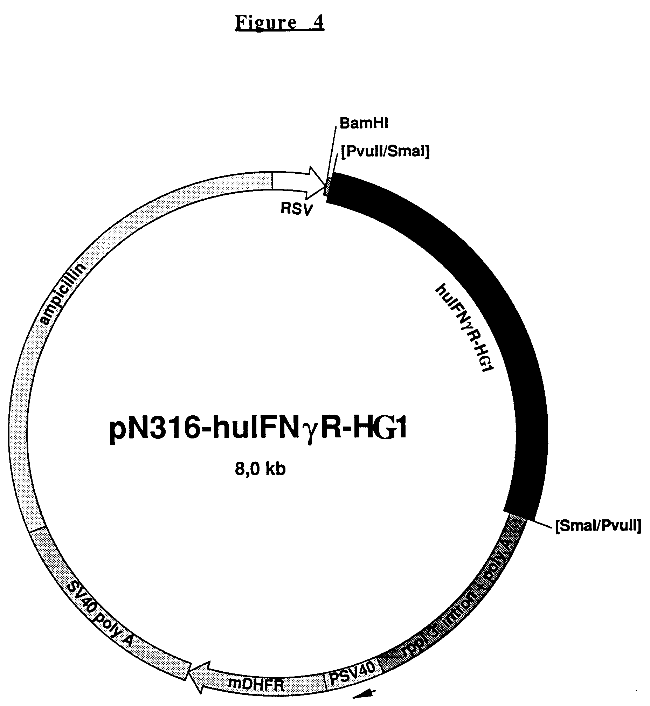

Plasmid phuIFNγR-HG1 was cleaved with SacI and KpnI enzymes. Then it was resuspended in an appropriate buffer and blunt-ended by Klenow Polymerase using standard procedures. The resulting fragment was purified and ligated into PvuII opened expression vector pN316. HindIII restriction enzyme digests of the resulting plasmids were performed and a construct harbouring approximately 2.55 and 0.95 kb fragments was selected. This construct was designated pN316-huIFNγR-HG1. A schematic drawing of this construct is shown in Fig. 4.

### Example 6

### Construction of a chimeric human IFNγR-IgG3 (HG3) molecule

The same protocol as described in Example 5 was used with the following exceptions:
The PCR 3' linker used was: 
5'-AATTCGAGCTC ACCTTTTATACTGCTATT GA (SEQ ID NO.5)
SacI
which creates one extra restriction site as indicated. The first 18 nucleotides downstream the SacI site match with the last 6 amino acids of soluble hIFNγR (positions 245 to 240 of the hIFNγR sequence, starting with Met as No. 1, as indicated in Aguet et al., supra). The last two nucleotides match with the 3^{rd} and 2^{nd} nucleotide of the codon for the amino acid in the position No. 239 of the hIFNγR.

Furthermore, a SacI digest was used instead of a SacI/HindIII double digest of the PCR product and pCD4-Hγ3 was used as a source of the immunoglobulin gene part. The pCD4-Hγ3 was digested with SacI and then the PCR fragment was ligated into the SacI digested pCD4-Hγ3 vector. A construct containing an approximately 7.2 Kb KpnI /SacII fragment was selected and designated phuIFNγR-HG3.

In addition, for ligation with expression vector pN316 the hIFNγR-IgG3 chimeric gene construct was digested with KpnI (which cleaves the DNA several nucleotides upstream of the SacI site which was originally introduced by the 5'-PCR primer; see Example 5 and SphI prior to endpolishing with T4 DNA Polymerase.

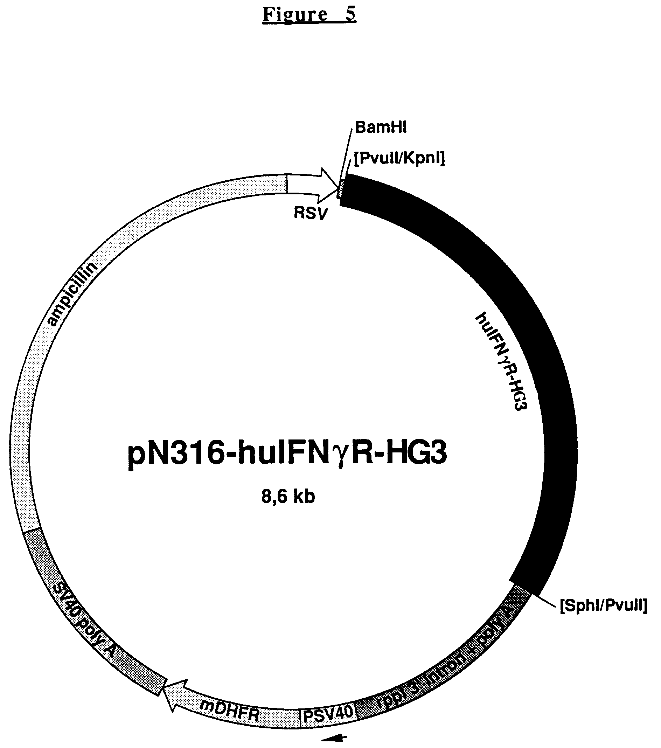

HindIII / SacII restriction enzyme double digests of the resulting plasmids were performed and a construct yielding a 2.3 kb fragment among other bands was selected. This contruct was designated pN316-huIFNγR-HG3. A schematic drawing of this construct is shown in Fig. 5.

### Example 7

### Construction of a chimeric human IFNγR-HCχ molecule

The same protocol as described in Example 5 was used with the following exceptions:
A human genomic λ-phage library prepared as described by Sambrook et al., supra was used as a source of the genomic immunoglobulin χ light chain constant region. A λ-clone containing the human immunoglobulin χ light chain gene was digested with HindIII and BamHI enzymes and a 5.4 kb fragment containing a genomic Cχ gene segment was purified and subsequently ligated into the Bluescript KS- plasmid (Stratagene, La Jolla, Ca.) generating plasmid pBS-HCχ.

The plasmid phuIFNγR-HG1 as described in Example 5 was used as a source for the hIFNγR fragment. This plasmid was opened at the SacI site and blunt ends were created using T4 DNA polymerase. XhoI recognition sites were added to the blunt ends by ligation of 2 synthetic palindromic oligonucleotides having the sequences: 
5'-CCGCTCGAGCGG (Seq. ID No.6)
3'-GGCGAGCTCGCC (Seq. ID No.7)
These oligonucleotides were obtained from Promega, Madison. The plasmids obtained after ligation of the XhoI recognition site containing the hIFNγR fragment was then digested with XhoI and HindIII.

The resulting fragment was gel-purified and subsequently ligated into XhoI/HindIII-opened plasmid pBS-HCχ described above generating plasmid phuIFNγR-HCχ.

Plasmid phuIFNγR-HCχ was digested with XhoI and BamHI thereby excising the hybrid gene construct of about 6,2 kb in length. This fragment was blunt ended and purified as described.

The resulting hIFNγR-HCχ chimeric gene construct was then ligated with PvuII-opened pN346 vector DNA yielding plasmid pN346-huIFNγR-HCₖ. A schematic drawing of this construct is shown in Fig. 6.

Selection of the plasmid constructs with preferred orientation of the insert was done as in Example 5, taking into account differences in the fragment length: the preferred construct being characterized by 0.85 and 6.8 kb, but not by 2.25 and 5.4 kb fragments (the remaining fragment being constant).

### Example 8

### Transfection of CHO-dhfr⁻cells and expression of chimeric hIFNγR-Ig polypeptides

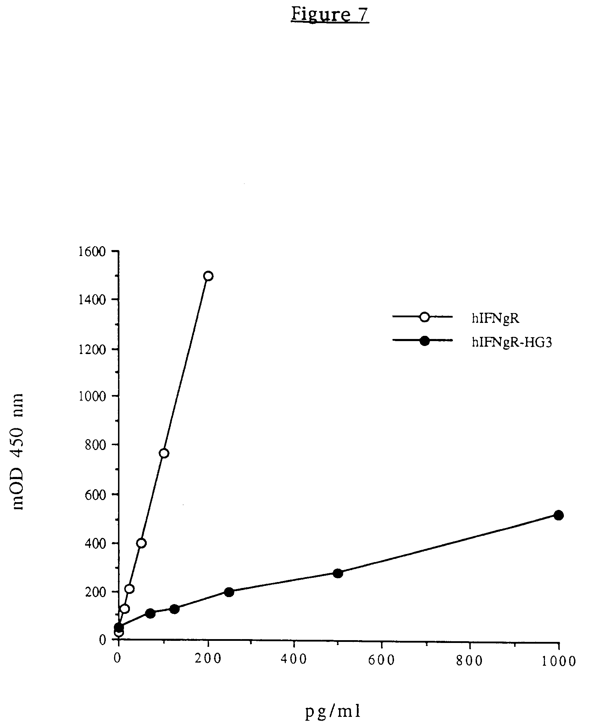

CHO-dhfr⁻cells were cultured in alpha-minimal essential medium (alpha - MEM, GIBCO/BRL, Paisley, Scotland) containing 5% Fetal Bovine Serum (FBS). The cells were plated into dishes (35 mm in diameter) to give a monolayer of approximately 90% confluence on the day of transfection. Prior to transfection the culture medium was aspirated and the monolayers washed with phosphate buffered saline (PBS), pH7,2, containing 0.14 M NaCl and 15 mM phosphate. One ml of alpha-MEM without FBS was added and the plates maintained at 37°C for three hours. The transfection mixture consisted of 10 µl Lipofectin (GIBCO, Gaithersburg, MD, U.S.A.), 1 µg pSV2-Neo plasmid DNA (Southern and Berg, J. Mol. Appl. Genet. 1, 327-341 [1982]) and 5 µg of either huIFNγR-HG1, huIFNγR-HG3 or huIFNγR-HCχ DNA. The mixtures were added to the cells and after 5 hours 1 ml of alpha-MEM containing 10% FBS was added to each dish. Then the cells were cultured for 24 hours. For selection, the cells were trypsinized and resuspended in selection medium consisting of alpha minus MEM (alpha-MEM missing nucleosides) and 1 mg/ml of G418 (GIBCOBRL, Paisley, Scotland) and plated into culture dishes (100 mm diameter). The dishes were incubated for 12 days. Well developed colonies were picked and transferred into 12-multiwell dishes. The supernatants of these clones were analyzed for the presence of the hIFNγR-Ig polypeptides by a specific ELISA (for details see Example 10, Figure 7 and Table I). The best producing clones were then amplified by successive passage in increasing concentrations of methotrexate (MTX, Sigma, St. Louis, MO), beginning at 0.01 µM up to 80 µM MTX. The yield of the secreted hIFNγR-Ig polypeptides reached the level of 10-20 µg/ml.

### Example 9

### Purification of the hIFNγR-Ig polypeptides

The hIFNγR-HG1 and huIFNγR-HG3 hybrid proteins were purified from supernatants of CHO cells using a protein G column (5 ml Protein G Sepharose 4 Fast Flow, Pharmacia LKB, Uppsala). Hybrid protein hIFNγR-HCₖ was purified using an anti-human IFNγR receptor column which had been produced by coupling 60 mg of monoclonal antibody γR46 to 3g CNBr activated Sepharose 4B (Pharmacia LKB, Uppsala) according to the manufacturer's instructions and packed into a column. Alternatively, hIFNγR-HCₖ was affinity purified on an anti-human χ Ig light chain affinity column produced as described above. Volumes of 2 liters supernatant were loaded at a flow rate of 50 ml per hour. The proteins were eluded with 0.1 M Glycin-HCl, pH2.8. Fractions of 1.5 ml were collected, analyzed by the ELISA for hIFNγR-Ig hybrid molecules and by SDS-PAGE under reduced conditions.

### Example 10

### Detection of hIFNγR-Ig polypeptides by ELISA

Supernatants of transfected CHO cells were assayed for the presence of the hIFNγR-Ig polypeptides by using the monoclonal antibodies γR46 and γR89 in an ELISA that detects hIFNγR. These monoclonal antibodies had been produced against the native human IFNγ receptor as described by Garotta et al., in J. Biol. Chem. 265, 6908-6915 [1990]. Both monoclonal antibodies bound only the non-reduced form of the human IFNγ receptor, recognized structural epitopes of the extracellular region of human IFNγ receptor and in- hibited IFNγ binding to cell-bound human IFNγ receptor. The antibody γR46 is an IgM that detects an epitope between amino acids 26-133 while the antibody γR89 is an IgG1 that reacts with another epitope located between amino acids 70-210. Monoclonal antibodies γR46 were affinity purified on an Anti-Mouse-IgM-Agarose column (Sigma, St.Louis, MO) according to the manufacturer's instructions. Monoclonal antibodies γR89 were affinity purified on protein G column (Pharmacia LKB, Uppsala) according to the manufacturer's instructions.

A stock solution of antibody γR46 was diluted with 0.1 M Naphosphate buffer, pH 6.5 to give 10 µg/ml of pure antibody solution. 100 µl of this solution containing 1 µg of pure antibody were distributed in Maxisorp microtiter plates (Nunc, Naperville, Il) to coat the flat-bottomed plastic wells. The coating reaction was performed overnight at 15-25°C. The residual binding capacity of plastic was then blocked by adding to each well 250 µl of blocking buffer (TRIS/HCl 0.2 M, pH 7.5) containing 10 mg/ml of Bovine serum albumine (BSA) and by incubating the microtiter plates at room temperature for 24 hours (h) at 15-25°C. The supernatants containing the hIFNγR-Ig polypeptides hIFNγR-HG3, hIFNγR-HGl or hIFNγR-HCχ expressed by transfected CHO cells, were diluted 1:5, 1:50, 1:500, 1:5000 with test buffer (TRIS/Acetate 0.05 M, pH 6.2) containing 5% Foetal Bovine serum (FSA). The standard solutions were diluted with test buffer to have 30 ng/ml, 10 ng/ml, 3 ng/ml, 1 ng/ml, 0.3 ng/ml and 0.1 ng/ml of soluble hIFNγR, hIFNγR-HG3, hIFNγR-HG1 or hIFNγR-HCχ. Before the test, the blocking buffer was poured off from the coated plates and a volume of 200 µl from each sample or standard dilution was distributed in 3 wells of coated plates. Finally, 50 µl (100 ng/ml) γR89 antibody conjugated with peroxidase (Gallati et al., J. Clin. Chem. Biochem. 20, 907-914, [1982]) were added to each well. After 16-24 h at 15-25°C, the plates were washed 6-8 times with deionized water and 200 µl of a tetramethylbenzidine/H₂O₂ solution (0.01M 3,3',5,5'-tetramethylbenzidine, 0.08 M H₂O₂ in 10% aceton, 90% ethanol) diluted 1:20 with substrate buffer (K-citrate, 0.03 M, pH 4.1) were added into each well. After 10 minutes at 15-25°C, the enzymatic reaction was stopped by adding 100 µl of 5% sulphuric acid to each well and the colour was then red at 540 nm wave length using a photometer (Titertek Multiskan MC, from Flow Laboratories, Woodcock Hill, U.K.). The concentration of hIFNγR-Ig polypeptides expressed by transfected CHO cells was determined on the basis of the proper standard curve with shIFNγR (for results see Table I below and Fig.7).

**Table I**

| DETERMINATION OF hIFNγR-Ig POLYPEPTIDE CONCENTRATION BY A SPECIFIC ELISA | | | | |
|---|---|---|---|---|
| Receptor protein | Sample | Reciprocal of Dilution | mOD 450 nm | µg/ml |
| hIFNγR-HG3(*) | 21 | 200 | 981 | 0.40 |
| | 22 | 1,000 | 1091 | 2.24 |
| | 23 | 5,000 | 578 | 5.60 |
| hIFNγR-HGl(*) | 11 | 200 | 862 | 3.48 |
| | 12 | 1,000 | 288 | 4.84 |
| | 13 | 5,000 | 822 | 8.27 |
| hIFNγR-HCχ(*) | 31 | 200 | 1271 | 5.28 |
| | 32 | 1,000 | 662 | 1.304 |
| | 33 | 5,000 | 1035 | 10.61 |
| shIFNγR(△) | 1 | 200 | 437 | 0.11 |
| | 2 | 1,000 | 598 | 0.78 |
| | 3 | 25,000 | 871 | 2.85 |

| | | | | |
|---|---|---|---|---|
| *) Data calculated on hIFNγR-HG3 standard curve | | | | |
| △) Data calculated on shIFNγR standard curve | | | | |

### Example 11

### Analysis of hIFNγR-Ig polypeptides by SDS-PAGE and Immunoblot

SDS-PAGE was performed on 7.5% gels. The samples were loaded in sample buffer with or without reducing agents for reducing or non-reducing SDS-PAGE, respectively. Bands were stained with Coomassie blue R-250 (Sigma, St.Louis, MO) or blotted to nitrocellulose and visualized by monoclonal antibodies γR46 or γR89 (Garotta et al., supra) and iodinated sheep Ig anti-mouse Ig (Amersham, Little Chalfont, U.K.) or by affinity purified anti human IFNγ receptor rabbit antibodies (Valente et al., Eur. J. Immunol. 22, 2403-2412 [1992]) and iodinated donkey Ig anti-rabbit Ig (Amersham, Little Chalfont, U.K.). The molecular weights of each of the hIFNγR-Ig polypeptides are summarized in Table II and III below.

### Example 12

### Inhibition of IFNγ binding to Raji cells by hIFNγR-Ig polypeptides

The binding capacity of the hIFNγR-Ig polypeptides expressed in CHO cells was determined by inhibition of IFNγ binding to Raji cell surface receptor. Solutions containing the hIFNγR-Ig polypeptides (hIFNγR-HG3, hIFNγR-HG1 and hIFNγR-HCₖ) were serially diluted with HBSS (Hank's basal salt solution; GIBCO/BRL, Paisley, U.K. and supplemented with 1% BSA and 15 mM HEPES) and 50 µl of each solution were distributed in triplicate into the wells of polystyrene U-bottomed microtiter plates (Costar, Cambridge, MA). 50 µl iodinated IFNγ 2ng (200 U) of a preparation with 2x10⁴ dpm/ng was added to each well after dilution in HBSS. Then 100 µl of HBSS medium containing 10⁶ Raji cells (ATCC CCL 86) were added. After incubation for 90 minutes at 0°C, the plates were centrifuged for 5 minutes at 100 x g, the supernatant with unbound radioactive IFNγ was discarded and the cells were washed twice with washing buffer (Hank's basal salt solution additioned with 0.1% BSA, 15 mM HEPES and 0.01% Triton X 100). Then the cells were resuspended in 200 µl washing buffer, transferred to flexible U-bottomed PVC microtiter plates (Dynatech, Chantilly, VA) and once more centrifuged. The flexible plates were cut and the radioactivity in each well was determined. IFNγ was iodinated using the chloramine T procedure (Greenwood et al., Biochem. J. 89, 114-123 [1963]).

Scatchard's analysis (Scatchard Ann. N.Y. Acad. Sci. 51, 660-672 [1949]) was performed with increasing doses (0.4-9 ng) of iodinated IFNγ, incubated with 10⁶ Raji cells. The amount of non-specific binding was determined in the presence of an excess of cold IFNγ (10 µg per ml). The capacity to inhibit the binding of IFNγ to Raji cells of the different receptor proteins is summarized in Table II below.

**Table II**

| Inhibition Of IFNγ Binding To Raji Cells | | | | |
|---|---|---|---|---|
| Receptor protein expressed | MW kDa | Binding sites | IC50 ng/ml | IC50 nM |
| hIFNγR-HG3 | 184 | 2 | 24 | 0.13 |
| hIFNγR-HG1 | 170 | 2 | 29 | 0.17 |
| hIFNγR-HCχ | 82 | 1 | 42 | 0.51 |
| shIFNγR | 31 | 1 | 100 | 3.23 |
| MW is the molecular mass evaluated by non-reducing SDS-PAGE. IC50 is the concentration that produces 50% inhibition of the binding of 3 ng of iodinated IFNγ to 10⁶ Raji cells. | | | | |

### Example 13

### Inhibition of the antiviral activity of IFNγ by hIFNγR-Ig polypeptides

The binding capacity of hIFNγR-Ig polypeptides expressed in CHO cells was determined by the inhibition of antiviral activity exerted by IFNγ. Solutions containing hIFNγR-Ig polypeptides (hIFNγR-HG3, hIFNγR-HG1 or hIFNγR-HCχ) were serially diluted with Minimal Essential Medium (MEM) from GIBCO/BRL supplemented with 5% FBS. 50 µl of each dilution were distributed in triplicate to wells of tissue culture grade flat-bottomed microtiter plates (Costar). Then 25 µl IFNγ solution containing 0.25 pg of IFNγ (1 U/ml of a preparation of IFNγ with a specific activity of 10⁸ U/mg) were added to each well. After 1 h at room temperature the IFNγ activity therein was determined by the cytopathic effect (CPE) reading method according to Armstrong (Methods in Enzymology, S. Pestka ed., Vol. 78, page 38, Academic Press New York [1981]). Accordingly, 50 µl of a WISH cell (ATCC CCL 25) suspension (4x10⁵ cells/ml) in 5% FBS-containing MEM were placed in each well of the 96-well microplates and incubation was conducted in a carbon dioxide gas incubator (5% CO2) at 37°C. After 24 h the culture supernatants were discarded and 100 µl of MEM medium with 2% FBS and 1000 plaque forming units of Encephalomyocarditis (EMC) Virus (ATCC VR-129B) were added to each well. The plates were incubated for 90 minutes at 37°C, the medium discarded and substituted with 100 µl 2% FBS-containing MEM. About 24 h later, when the cells in wells not containing IFNγ show 100% CPE, the medium was discarded and the remaining adherent cells were washed with PBS. Then 100 µl of a crystal violet solution (1% crystal violet, 10% ethanol in water) were added to each well. After 5 minutes, the unbound dye was removed by washing with water before the stained monolayers were dried. Finally, the dye associated with the cells was extracted in 100 µl methylcellosolve and quantitated by reading the absorbance at 550 nm in a Titertek Multiskan MC (Flow Laboratories, Woodcock Hill, U.K.). Since the amount of dye is proportional to the number of cells present per well, the obtained absorbance readings are a measure for the protection of IFNγ against the viral cytopatic effect. The capacity of the hIFNγR-Ig polypeptides hIFNγR-HG3, hIFNγR-HG1 or hIFNγR-HCχ to inhibit the antiviral activity exerted by IFNγ is summarized in Table III below.

**Table III**

| Inhibition Of IFNγ Antiviral Activity | | | | |
|---|---|---|---|---|
| Receptor protein expressed | MW kDa | Binding sites | IC50 µg/ml | IC50 nM |
| hIFNγR-HG3 | 184 | 2 | 2.0 | 11 |
| hIFNγR-HG1 | 170 | 2 | 4.4 | 26 |
| hIFNγR-HCχ | 82 | 1 | nd | nd |
| shIFNγR | 31 | 1 | 6.7 | 216 |
| MW is the molecular mass evaluated by non-reducing SDS-PAGE IC50 is the concentration that produces 50% inhibition of antiviral activity exerted by 10 U/ml (5 ng/ml) of IFNγ. nd = not done | | | | |

## Claims

1. A DNA sequence comprising a combination of two partial DNA sequences, one partial sequence coding for a fragment of the human interferon-γ receptor which fragment binds human interferon-γ and the other partial sequence coding for part or all of the constant domains of human immunoglobulin heavy or light chains such as IgG, IgA, IgM, IgE or Cχ.

2. A DNA sequence according to claim 1, wherein one partial sequence codes for a fragment of the human interferon-γ receptor which binds human interferon-γ and the other partial sequence codes for part or all of the constant domain of IgG, preferably IgG1 and IgG3, or for part or all of the constant domain of Cχ.

3. A vector comprising a DNA sequence as claimed in claim 1 or 2.

4. A vector as claimed in claim 3 capable of directing expression in a compatible prokaryotic, insect or mammalian host cell.

5. A prokaryotic, mammalian or insect host cell transformed with a vector as claimed in claim 3 or 4.

6. A recombinant protein coded for by a DNA sequence as claimed in claim 1 or 2.

7. A recombinant protein as claimed in claim 6 selected from the group consisting of hIFNγR-HG1, hIFNγR-HG3 or hIFNγR-HCχ.

8. A process for the production of a protein as claimed in claim 6 or 7 which process comprises cultivating a transformed host as claimed in claim 5 in a suitable medium and isolating said protein.

9. A pharmaceutical composition which contains one or more compounds according to claim 6 or 7, if desired in combination with additional pharmaceutically active agents and pharmaceutically acceptable carrier materials.

10. The use of a compound according to claim 6 or 7 for the preparation of pharmaceutical compositions.

11. The use of a compound according to claim 6 or 7 for the preparation of a medicament for the treatment of illnesses especially autoimmune diseases, chronic inflammations, delayed hypersensitivity, allotransplant rejections, multiple sclerosis and fulminant hepatitis, inflammatory neurological diseases, and neurological complications of AIDS, poliovirus infections, lyme disease and septicemia.
